# EUROPEAN PATENT APPLICATION

(11) **EP 1 637 072 A1**
(43) Date of publication of application: **22.03.2006**
(21) Application number: 05019794.6
(22) Date of filing: 12.09.2005
(51) Int. Cl.: A61B 5/022

(54) **Cuff for blood pressure monitor**

(30) Priority: 15.09.2004 JP 2004268514
(71) Applicant: Omron Healthcare Co., Ltd., Kyoto 615-0084 (JP)
(72) Inventor: Kishimoto, Hiroshi Omron Healthcare Co., Ltd., Ukyo-ku Kyoto-shi Kyoto 615-0084 (JP); Sano, Yoshihiko Omron Healthcare Co., Ltd., Ukyo-ku Kyoto-shi Kyoto 615-0084 (JP); Karo, Hiromichi Omron Healthcare Co., Ltd., Ukyo-ku Kyoto-shi Kyoto 615-0084 (JP)
(74) Representative: Kilian, Helmut

(57) **Abstract**

A cuff (1A) for a blood pressure monitor is wound around a measurement site of the living body, and includes an air bag (10A) having an inflated/deflated space (15) formed therein by melting and bonding rims of an outer wall portion (11a) and an inner wall portion (11b) together. The air bag (10A) contains a band-shaped connecting portion (12), which has ends respectively attached to one end (B) and the other end (C) of the air bag (10A) in its winding direction (A) around the living body, so as to extend substantially continuously from the one end (B) to the other end (C) of the air bag (10A). The band-shaped connecting portion (12) is arranged inside the inflated/deflated space (15) of the air bag (10A). With this configuration, a cuff for a blood pressure monitor permitting uniform inflation of the air bag over the entire region when the pressurized air is introduced therein is obtained.

## Description

### BACKGROUND OF THE INVENTION

### Field of the Invention

The present invention relates to a cuff for a blood pressure monitor wound around a measurement site of a living body, such as a wrist, an upper arm or the like, for measurement of blood pressure values.

### Description of the Background Art

To measure a blood pressure value, generally, a cuff provided with a fluid bag for pressing an artery located within a living body is wound around the body surface, and arterial pressure pulse waves caused in the artery by inflation/deflation of the fluid bag are detected to measure the blood pressure value. Here, the cuff refers to a band-shaped structure having a bladder, which can be wound around a part of a living body, for use in measurement of arterial pressure of an upper limb, a lower limb or the like by introducing fluid such as gas or liquid into the bladder. Thus, the cuff represents the concept including the fluid bag as well as members for winding the fluid bag around the living body. Particularly, the cuff wound around and fitted on an arm is also called an arm band or a manchette.

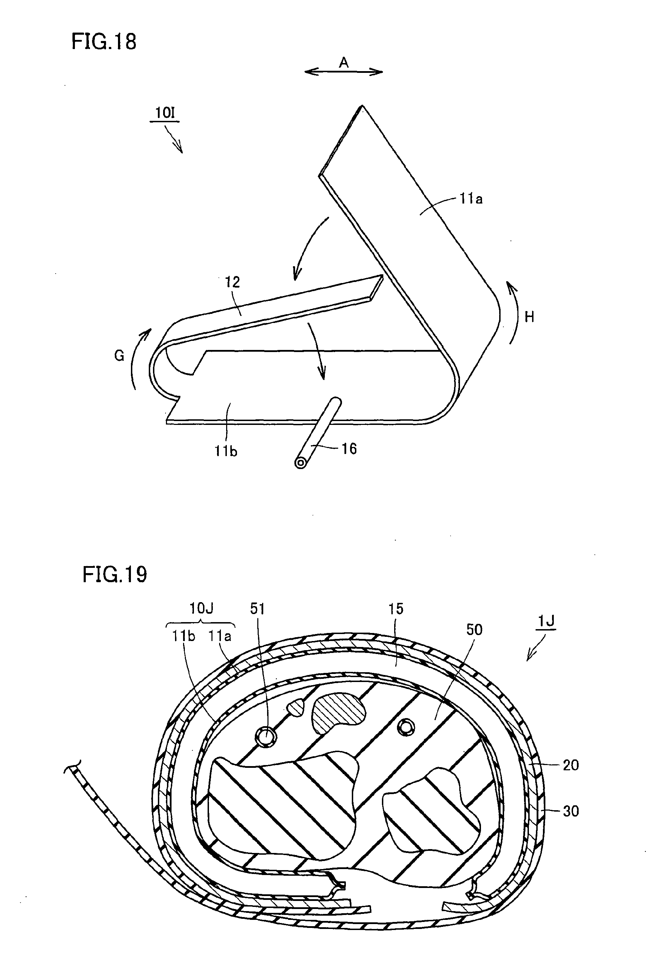

Fig. 19 is a schematic cross sectional view showing a structure of a conventional cuff for a blood pressure monitor, illustrating the state where the cuff is wound around a wrist that is the measurement site. As shown in Fig. 19, the cuff 1J for a blood pressure monitor of the conventional example includes, among others, an air bag 10J that is a fluid bag, a curled elastic member 20 that is an elastic member located on the outside of air bag 10J and wound in an approximately cylindrical shape and changeable in size in a radial direction, and a fastening band 30 for securing the cuff to the living body.

As shown in Fig. 19, in the state where cuff 1J is wound around the measurement site of wrist 50, cuff 1J is secured to the wrist in an immovable manner by means of a securing member (not shown) such as a velcro fastener provided at fastening band 30. As such, air bag 10J is positioned between wrist 50 and curled elastic member 20 that is arranged inside fastening band 30.

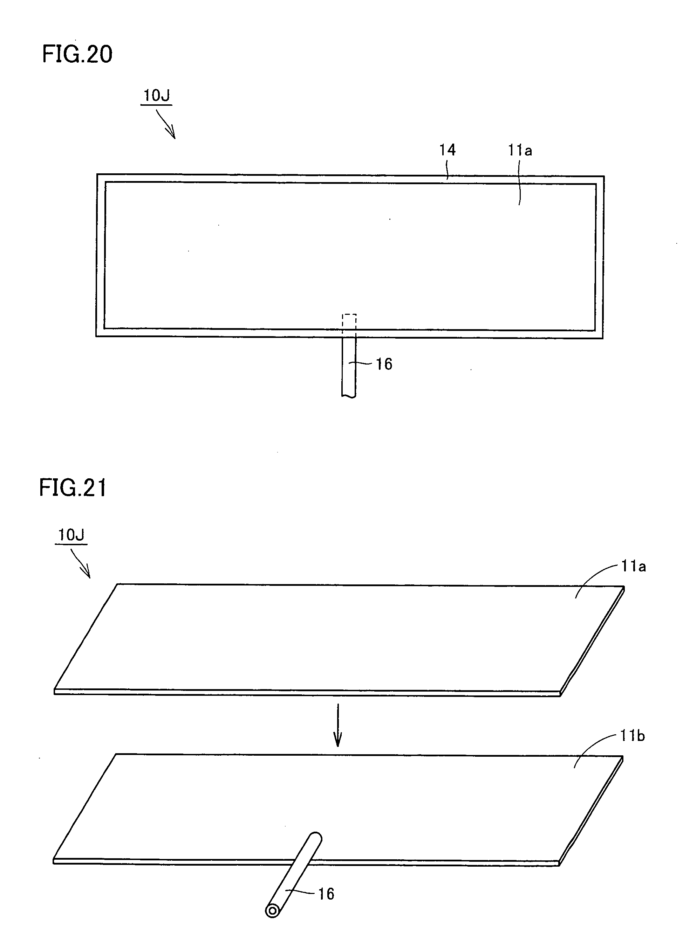

Fig. 20 is a schematic top plan view showing a structure of an air bag of the conventional cuff for a blood pressure monitor shown in Fig. 19. Fig. 21 is an exploded perspective view of the air bag in Fig. 20. As shown in Fig. 20, air bag 10J has an approximately rectangular shape in two dimensions, and has an inflated/deflated space 15 (see Fig. 19) that is a hollow space formed therein. A tube 16 is connected to this inflated/deflated space 15, to let the air in/out through tube 16. As shown in Fig. 21, air bag 10J having the above-described configuration is formed by laying one on another an outer wall portion 11a and an inner wall portion 11 b that are formed of two resin sheets of an approximately rectangular shape in two dimensions, which are melted and bonded together along the four sides. As such, a bonded portion 14 is formed along the rim of air bag 10J, as shown in Fig. 20, making inflated/deflated space 15 formed inside air bag 10J in an airtight manner.

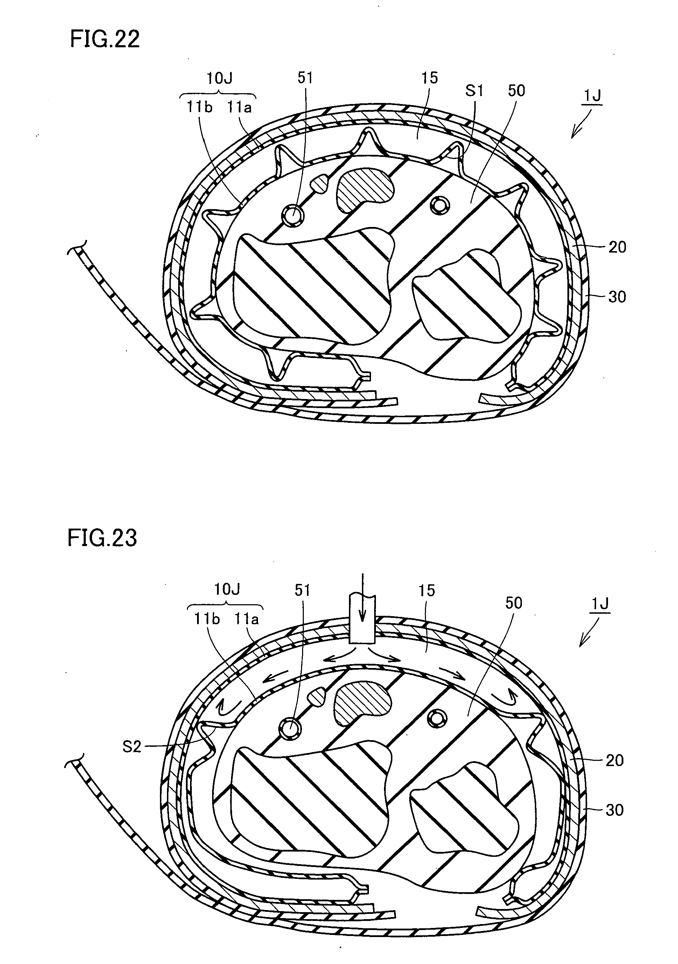

Fig. 22 is a schematic cross sectional view showing the fitted state of the cuff for a blood pressure monitor in Fig. 19 at the time of measurement of the blood pressure values. As shown in Fig. 22, in the fitted state of the cuff shown in Fig. 19, the pressurized air is introduced into air bag 10J, to make it inflated between curled elastic member 20 and wrist 50. With this inflation of air bag 10J, an artery 51 located under the skin of wrist 50 is pressed by air bag 10J, to enable measurement of the blood pressure values.

To enable accurate measurement of the blood pressure values, it is necessary for air bag 10J to be inflated uniformly over the entire region when the pressurized air is introduced therein. The problem at this time is occurrence of wrinkles S 1 at the surface of air bag 10J inflated by the pressurized air.

Wrinkles S1 are caused when air bag 10J is inflated. Of outer wall portion 11a and inner wall portion 11b constituting the bag-shaped portion of air bag 10J, inner wall portion 11b located inside is decreased in diameter, and the redundant part that cannot escape anywhere gathers locally. Wrinkles S 1 occur mostly in the direction crossing the winding direction of cuff 1J around the living body. Occurrence of wrinkles S1 leads to degradation of avascularization performance by air bag 10J, disadvantageously decreasing the accuracy in measurement of blood pressure values. Particularly, in the case where large wrinkles S 1 occur in the proximity of artery 51, as shown in Fig. 22, air bag 10J cannot sufficiently press artery 51 located beneath the skin, causing considerable errors in the blood pressure values measured.

Fig. 23 is a schematic cross sectional view illustrating the problem in the case where wrinkles S 1 occurred are deeper and larger, and further increase in size to cause bending S2 of air bag 10J. As shown in Fig. 23, when bigger and deeper wrinkles S1 occur and increase in size to cause bending S2 of air bag 10J, and bending S2 extends from one end to the other end of air bag 10J in the width direction of cuff 1J (i.e., in the direction approximately parallel to artery 51 in the fitted state of the cuff), then this bending S2 would block the flow of the air (shown by arrows in Fig. 23) being introduced into air bag 10J. In this case, it will not be possible to sufficiently inflate air bag 10J over the entire region, and thus, the blood pressure values measured will include considerable errors.

Japanese Patent Laying-Open Nos. 62-072315 and 2003-038451 disclose techniques for preventing the problems caused by occurrence of wrinkles. According to the technique disclosed in Japanese Patent Laying-Open No. 62-072315, to prevent blood stasis due to wrinkles caused in the fluid bag, a plurality of joint potions for joining one surface on the inner peripheral side and the other surface on the outer peripheral side of the fluid bag are arranged in the longitudinal direction of the fluid bag, so as to intentionally localize occurrence of wrinkles in the air bag in prescribed locations. According to the technique disclosed in Japanese Patent Laying-Open No. 2003-038451, to prevent decrease of accuracy in measurement because of degradation of avascularization performance due to occurrence of wrinkles, cushion members are provided inside the air bag intermittently such that the air bag is maintained in the inflated state before the pressurized air is introduced therein. This localizes the wrinkles caused in the air bag between the cushion members provided intermittently, to thereby prevent occurrence of wrinkles or bending in the portions provided with the cushion members.

With the above-described techniques, however, wrinkles are caused to occur locally in prescribed positions of the air bag, which may rather increase the probability of occurrence of bending in the relevant positions. If such bending occurs in the air bag, the above-described flow of the air in the inflated/deflated space will be blocked, making it difficult to uniformly inflate the air bag over the entire region. In such a case, it is not possible to uniformly and stably press the measurement site, leading to occurrence of measurement errors.

Further, Japanese Patent Laying-Open No. 2004-159967 discloses a technique to prevent bending of the air bag. According to the technique disclosed in Japanese Patent Laying-Open No. 2004-159967, a double-cuff for measuring blood pressure is provided with an air bag for avascularization and an air bag for detecting pulse waves. A core member harder than the air bag for detecting pulse waves is arranged inside the air bag for detecting pulse waves, to prevent bending of the air bag for detecting pulse waves during the fitting operation.

This technique, however, is directed to prevent bending of a small-sized air bag for detecting pulse waves that is arranged only in a specific part between the air bag for avascularization and the measurement site of the living body in the cuff-fitted state. The technique is not for preventing bending of a large-sized air bag for avascularization that is fitted to surround the measurement site. Accordingly, the above-described technique is effective only when the air bag for pressing the living body is formed of separate bags for avascularization and for detection of pulse waves. It does not function effectively for the cuff configured with one air bag that is used both for avascularization and for detection of pulse waves. In other words, in the case of a cuff configured to use one air bag for both avascularization and pulse wave detection, it is necessary to wind the cuff around the living body such that the air bag surrounds the living body in the circumferential direction including the measurement site. Thus, provision of a hard core member inside the air bag would considerably adversely affect the fitting of the cuff.

Further, although the above-described technique may prevent bending of a small-sized air bag for detecting pulse waves, it would not be able to suppress occurrence of large wrinkles or bending of a large-sized air bag for avascularization caused when the pressurized air is introduced therein. Thus, in the case where big wrinkles or bending occur locally in the air bag for avascularization, it is not possible to uniformly press the measurement site over the entire region, resulting in degradation in avascularization performance. Accordingly, the problem of degradation of accuracy in measurement of the blood pressure values is yet to be solved.

### SUMMARY OF THE INVENTION

An object of the present invention is to provide a cuff for a blood pressure monitor permitting a fluid bag to be inflated uniformly through the entire region when the pressurized air is introduced therein.

A cuff for a blood pressure monitor according to the present invention is wound around a living body, and includes a fluid bag having an inflated/deflated space permitting a fluid to come in/out formed therein by joining rims of sheet-shaped members laid one on another. The fluid bag includes a band-shaped connecting portion, which is arranged inside the inflated/deflated space, extends substantially continuously from one end to the other end of the fluid bag in a winding direction along which the fluid bag is wound around the living body, and has its ends respectively attached to the one end and the other end of the fluid bag.

With this configuration, passages for the air to flow therethrough are formed on the outside of the respective ends in the width direction of the band-shaped connecting portion that is arranged inside the inflated/deflated space of the air bag. Thus, when the pressurized air is introduced into the air bag, the air passes through the passages and flows uniformly from the one end to the other end in the winding direction of the air bag, enabling uniform inflation of the fluid bag over the entire region. Accordingly, it is possible to press the measurement site of the living body over the entire region, ensuring accurate and stable measurement of blood pressure values. Herein, the winding direction of the fluid bag with respect to the living body refers to the direction in which the fluid bag being wound around the living body extends in the fitted state, which coincides with the circumferential direction of the cuff wound around the living body in the fitted state.

In the above-described cuff for a blood pressure monitor of the present invention, preferably, a plurality of such band-shaped connecting portions are arranged inside the inflated/deflated space.

With this configuration, the cross sectional area of the air passages formed on the outside of the respective ends of the band-shaped connecting portion can be increased, or the number of such air passages in the inflated/deflated space can be increased. This further ensures uniform inflation of the air bag over the entire region.

Preferably, in the above-described cuff for a blood pressure monitor of the present invention, the sheet-shaped members and the band-shaped connecting portion are formed of resin materials that can be melted and bonded together. In this case, joining of the rims of the sheet-shaped members and attaching of the band-shaped connecting portion to the fluid bag are preferably carried out by melting and bonding the rims of the sheet-shaped members and the band-shaped connecting portion together in an integral manner.

Still preferably, in the above-described cuff for a blood pressure monitor of the present invention, the sheet-shaped members and the band-shaped connecting portion are formed of a same resin material. In this case, joining of the rims of the sheet-shaped members and attaching of the band-shaped connecting portion to the fluid bag are preferably carried out by melting and bonding the rims of the sheet-shaped members and the band-shaped connecting portion together in an integral manner.

With this configuration, it is readily possible to fabricate the air bag containing the band-shaped connecting portion in a single melting and bonding operation. Accordingly, a cuff for a blood pressure monitor exhibiting excellent avascularization performance can be provided in a simple and inexpensive manner.

Preferably, in the above-described cuff for a blood pressure monitor of the present invention, the fluid bag includes an inner wall portion positioned on an inner side and an outer wall portion positioned on an outer side in a fitted state of the cuff. The sheet-shaped members are configured with a first resin sheet having the band-shaped connecting portion and one of the inner wall portion and the outer wall portion, and a second resin sheet having the other of the inner wall portion and the outer wall portion, and the fluid bag is formed by folding the first resin sheet, laying the first resin sheet on the second resin sheet, and by melting and bonding rims of the first and second resin sheets together.

Still preferably, in the above-described cuff for a blood pressure monitor of the present invention, the fluid bag includes an inner wall portion positioned on an inner side and an outer wall portion positioned on an outer side in a fitted state of the cuff. The sheet-shaped members are configured with one resin sheet having the inner wall portion, the outer wall portion and the band-shaped connecting portion, and the fluid bag is formed by folding the resin sheet such that the inner and outer wall portions are laid one on another, with the band-shaped connecting portion arranged therebetween, and by melting and bonding rims of the inner and outer wall portions of the resin sheet together.

With this configuration, it is readily possible to fabricate the air bag containing the band-shaped connecting portion with one or two resin sheets. Accordingly, a cuff for a blood pressure monitor having excellent avascularization performance can be provided in a simple and inexpensive manner.

According to the present invention, the air bag can be inflated uniformly over the entire region when the pressurized air is introduced therein. This realizes stable avascularization performance at the time of measurement, and accordingly, accurate and stable measurement of the blood pressure values is ensured.

The foregoing and other objects, features, aspects and advantages of the present invention will become more apparent from the following detailed description of the present invention when taken in conjunction with the accompanying drawings.

### BRIEF DESCRIPTION OF THE DRAWINGS

Fig. 1 is a schematic cross sectional view showing a structure of a cuff for a blood pressure monitor according to a first embodiment of the present invention, illustrating the state where the cuff for a blood pressure monitor is wound around a wrist that is a measurement site.
Fig. 2 is a schematic top plan view showing a structure of an air bag of the cuff for a blood pressure monitor according to the first embodiment.
Figs. 3 and 4 are schematic cross sectional views of the air bag shown in Fig. 2, taken along the line III-III and the line IV-IV, respectively, in Fig. 2.
Fig. 5 is an exploded perspective view of the air bag shown in Figs. 2-4.
Fig. 6 is a schematic cross sectional view showing the state where the cuff for a blood pressure monitor of the first embodiment is wound for measurement of the blood pressure values.
Fig. 7 is a schematic cross sectional view of an air bag of a cuff for a blood pressure monitor according to a second embodiment of the present invention, taken along the winding direction of the cuff.
Fig. 8 is a schematic cross sectional view of the air bag of the cuff for a blood pressure monitor of the second embodiment, taken along the direction orthogonal to the winding direction of the cuff.
Fig. 9 is an exploded perspective view of the air bag of the cuff for a blood pressure monitor of the second embodiment.
Figs. 10 and 11 show modifications of the cuff for a blood pressure monitor of the second embodiment, specifically showing the cross sectional views of the air bags taken along the direction orthogonal to the cuff winding direction.
Fig. 12 is a schematic top plan view showing a structure of an air bag of a cuff for a blood pressure monitor according to a third embodiment of the present invention.
Fig. 13 is an exploded perspective view of the air bag of the cuff for a blood pressure monitor of the third embodiment.
Fig. 14 is a schematic top plan view showing a structure of an air bag of a cuff for a blood pressure monitor according to a fourth embodiment of the present invention.
Fig. 15 is an exploded perspective view of the air bag of the cuff for a blood pressure monitor of the fourth embodiment.
Figs. 16-18 are exploded perspective views of air bags of cuffs for a blood pressure monitor according to fifth through seventh embodiments of the present invention.
Fig. 19 is a schematic cross sectional view showing a structure of a conventional cuff for a blood pressure monitor, illustrating the state where the cuff for a blood pressure monitor is wound around a wrist that is the measurement site.
Fig. 20 is a schematic top plan view showing a structure of an air bag of the conventional cuff for a blood pressure monitor shown in Fig. 19.
Fig. 21 is an exploded perspective view of the air bag shown in Fig. 20.
Fig. 22 is a schematic cross sectional view of the conventional cuff for a blood pressure monitor shown in Fig. 19 in the fitted state at the time of measurement.
Fig. 23 is a schematic cross sectional view of the conventional cuff for a blood pressure monitor shown in Fig. 19, showing the case where wrinkles occurred in the air bag have increased in size and depth to cause bending.

### DESCRIPTION OF THE PREFERRED EMBODIMENTS

Hereinafter, embodiments of the present invention will be described with reference to the drawings. In the following embodiments, a cuff for use in a wrist blood pressure monitor using the wrist as the measurement site for measurement of blood pressure values will be explained by way of example.

### First Embodiment

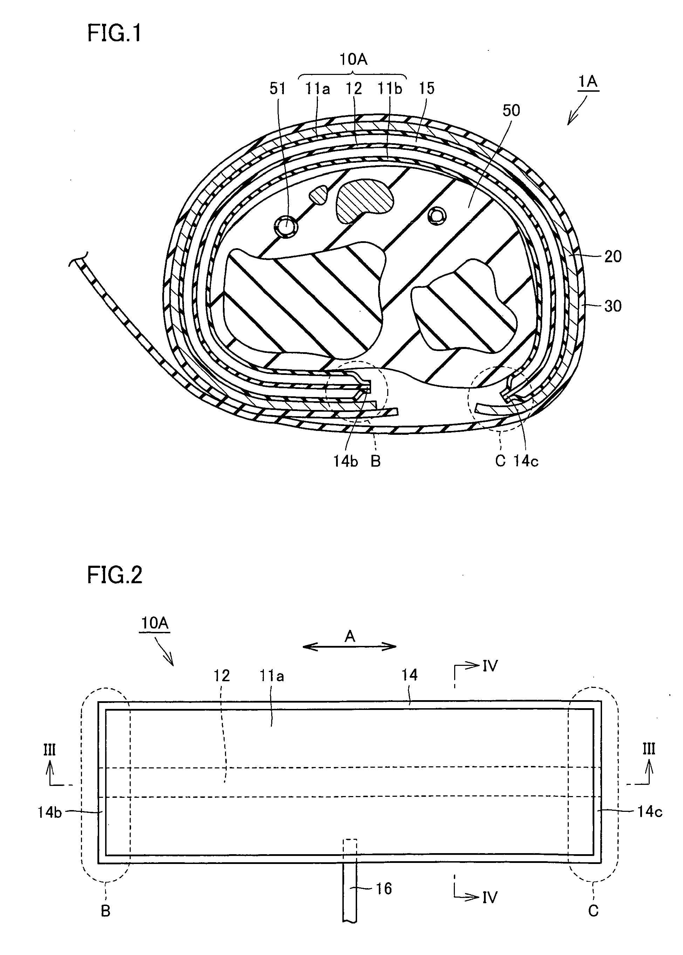

Fig. 1 is a schematic cross sectional view of a cuff for a blood pressure monitor according to a first embodiment of the present invention, showing the state where the cuff is wound around a wrist. As shown in Fig. 1, the cuff 1A for a blood pressure monitor of the present embodiment includes, among others, an air bag 10A that is a fluid bag, a curled elastic member 20 that is an elastic member located on the outside of air bag 10A and wound in an approximately cylindrical shape radially changeable in size, and a fastening band 30 for securing cuff 1A to the living body. Air bag 10A has its outer peripheral surface fixed to the inner peripheral surface of curled elastic member 20. The outer peripheral surface of curled elastic member 20 is fixed to the inner peripheral surface of fastening band 30.

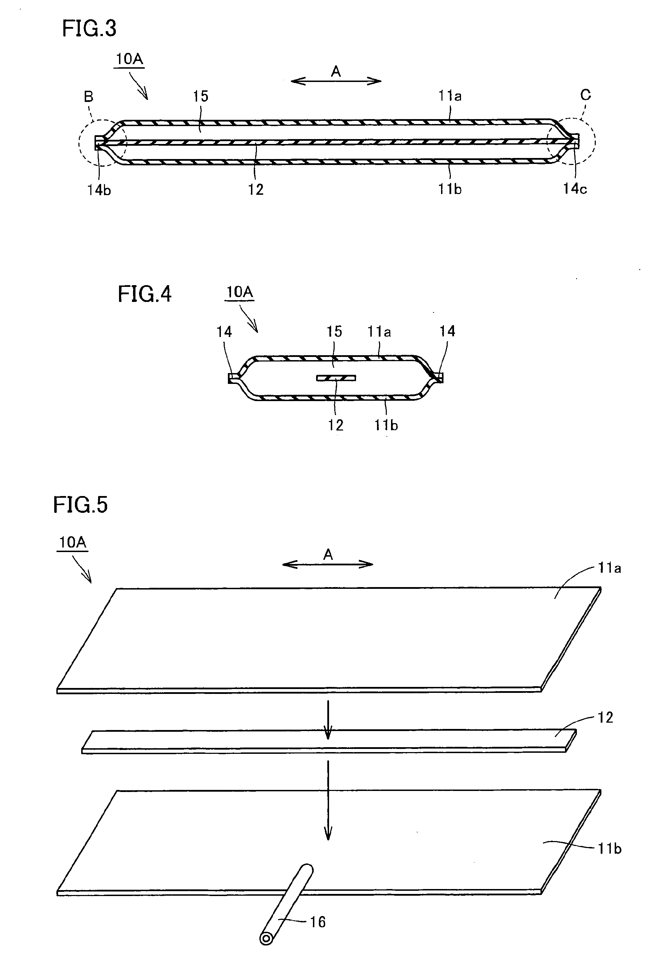

Fig. 2 is a schematic top plan view showing the air bag in Fig. 1 in a spread state. Fig. 3 is a schematic cross sectional view of the air bag in Fig. 2, taken along the line III-III therein, and Fig. 4 is a schematic cross sectional view of the air bag of Fig. 2, taken along the line IV-IV therein. As shown in Figs. 2-4, the air bag 10A of cuff 1A for a blood pressure monitor of the present embodiment is a bag-shaped member of an approximately rectangular shape in two dimensions in the spread state, and is provided with an outer wall portion 11a located on the side of curled elastic member 20 in the fitted state, an inner wall portion 11b located on the wrist side, and a band-shaped connecting portion 12 located therebetween.

As shown in Figs. 2-4, outer wall portion 11a and inner wall portion 11b are made of resin sheets identified as sheet-shaped members. The two resin sheets are laid one on another and their four sides are melted and bonded together to form the bag-shaped portion of air bag 10A. As such, air bag 10A has a bonded portion 14 along the rim. Outer wall portion 11a and inner wall portion 11b forming the bag-shaped portion of air bag 10A extend in the winding direction of cuff 1A on the wrist. Herein, the winding direction on or around the wrist refers to the direction in which air bag 10A wound around the wrist extends in the fitted state of the cuff. Thus, it corresponds to the circumferential direction of cuff 1A applied to surround the wrist in the fitted state. Normally, cuff 1A is formed to be elongated in the winding direction around the wrist, and thus, the longitudinal direction of air bag 10A coincides with the winding direction around the wrist. In the spread state of air bag 10A, this winding direction around the wrist corresponds to the direction shown by an arrow A in Figs. 2 and 3.

As shown in Figs. 3 and 4, an inflated/deflated space 15 that is a hollow space is located inside air bag 10A. As shown in Fig. 2, a tube 16 is connected to inflated/deflated space 15 on one end, and the other end of tube 16 is connected to an air system such as a pressurizing pump provided in the main unit of the blood pressure monitor (not shown). With the function of the air system, the air that is a fluid enters and leaves the inflated/deflated space 15 that is the internal space of air bag 10A. Particularly, when the compressed air is introduced into air bag 10A in the state where cuff 1A is fitted to the wrist, inflated/deflated space 15 is inflated, causing air bag 10A to be deformed to increase its thickness in the radial direction.

Inflated/deflated space 15 contains band-shaped connecting portion 12 therein. Band-shaped connecting portion 12 is formed of a band-shaped resin sheet that extends in the direction shown by arrow A in the figure that corresponds to the winding direction of air bag 10A. Band-shaped connecting portion 12 has ends in its extending direction attached respectively to one end B and the other end C of air bag 10A. More specifically, the ends of band-shaped connecting portion 12 are melted and bonded to outer wall portion 11a and inner wall portion 11b in bonded portions 14b and 14c that constitute the opposite sides of bonded portion 14 in the winding direction of cuff 1A. As shown in Figs. 2 and 4, band-shaped connecting portion 12 is formed to have a width narrower than those of outer wall portion 11a and inner wall portion 11b, and is located in inflated/deflated space 15 at an approximately central portion in the width direction of air bag 10A.

For the resin sheets constituting outer wall portion 11a and inner wall portion 11b, any material can be used as long as it is highly stretchable, permits melting and bonding of the sheets, and suppresses leakage of the air from inflated/deflated space 15 after such melting and bonding. From these standpoints, optimal materials for the resin sheets include, e.g., copolymer of ethylene-vinyl acetate (EVA), soft polyvinyl chloride (PVC), polyurethane (PU), crude rubber, and the like.

As for the material of the resin sheet constituting band-shaped connecting portion 12, any material can be employed as long as it is highly stretchable and permits melting and bonding together with the resin sheets constituting outer and inner wall portions 11a and 11b. Preferably, the same material as that of outer and inner wall portions 11a and 11b is used for band-shaped connecting portion 12.

Fig. 5 is an exploded perspective view of the air bag shown in Figs. 2-4. As shown in Fig. 5, in fabricating air bag 10A, a narrow resin sheet constituting band-shaped connecting portion 12 is laid in place between two wide resin sheets constituting outer wall portion 11a and inner wall portion 11 b, and the three resin sheets are melted and bonded together at a pair of opposite sides located at the ends in the winding direction of air bag 10A. The two sheets constituting outer wall portion 11a and inner wall portion 11b are melted and bonded together at the remaining opposite sides, with a tube 16 being inserted between the resin sheets at one side thereof. As such, the three resin sheets constituting outer wall portion 11a, inner wall portion 11b and band-shaped connecting portion 12 are melted and bonded together in a single operation, to thereby form air bag 10A in an integral manner.

Hereinafter, the fitted state where the cuff 1A for a blood pressure monitor provided with air bag 10A having the above-described configuration is wound around the wrist will be described. As shown in Fig. 1, in the state where cuff 1A is wound around wrist 50, cuff 1A is secured to wrist 50 in an immovable manner by means of a securing member (not shown) such as a velcro fastener provided on fastening band 30. This permits air bag 10A to be wound and positioned between curled elastic member 20 inside fastening band 30 and wrist 50. In this state, there are no wrinkles noticeable on the surface of air bag 10A, since the compressed air is yet to be introduced therein.

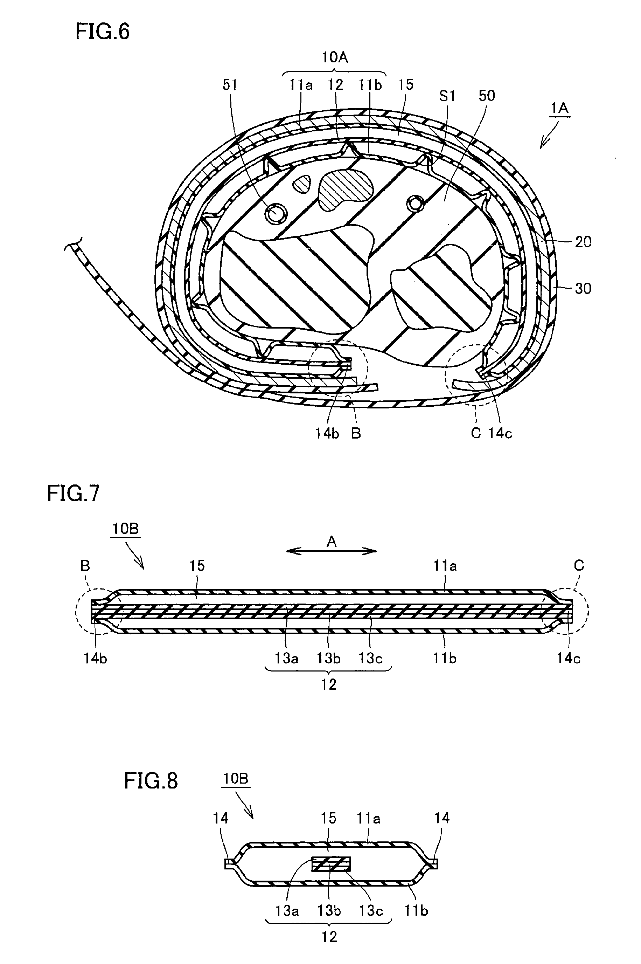

Next, the measurement state where the compressed air is introduced into air bag 10A in the fitted state shown in Fig. 1 will be described. Fig. 6 is a schematic cross sectional view showing the wound state of the cuff for a blood pressure monitor of Fig. 1 at the time of measurement. As shown in Fig. 6, when the compressed air is introduced into air bag 10A in the fitted state in Fig. 1, air bag 10A is inflated between curled elastic member 20 and wrist 50. Correspondingly, of outer wall portion 11a and inner wall portion 11b constituting air bag 10A, inner wall portion 11b located inside comes to suffer wrinkles S 1. Wrinkles S 1 are caused as inner wall portion 11b is reduced in diameter due to inflation of the bag-shaped portion of air bag 10A, and the redundant part that cannot escape anywhere gathers locally.

As described above, in the cuff 1A for a blood pressure monitor according to the present embodiment, band-shaped connecting portion 12 is located inside inflated/deflated space 15 of air bag 10A, continuously extending from one end B to the other end C in the winding direction of air bag 10A. Thus, in the fitted state of the cuff, passages through which the air introduced into air bag 10A can flow are secured on the outside of the respective ends in the width direction of band-shaped connecting portion 12. In other words, with provision of band-shaped connecting portion 12 having a certain thickness, even in the positions suffering wrinkles S1, close contact between outer wall portion 11a and inner wall portion 11b of air bag 10A can be prevented, and the air passages will not be blocked even if wrinkles S 1 increase in size. This ensures that the air introduced into inflated/deflated space 15 flows uniformly in the winding direction of air bag 10A. Accordingly, uniform inflation of air bag 10A over the entire region is ensured, and uneven inflation of the air bag due to the bending of the air bag that would occur in a conventional cuff for a blood pressure monitor is suppressed. As a result, stable avascularization performance is achieved during avascularization, and accurate and stable measurement of blood pressure values is enabled.

Further, with the function of the air passages formed by arranging band-shaped connecting portion 12 in inflated/deflated space 15, air bag 10A is pressurized uniformly over the entire region, so that wrinkles S1 having occurred are prevented from increasing in size. This causes fine wrinkles to be distributed over the entire region of air bag 10A, thereby enabling uniform pressing of the surface of wrist 50 over the entire region in the circumferential direction. As a result, accurate and stable measurement of blood pressure values is ensured. Further, occurrence of blood stasis due to the tightening of the cuff around the wrist can be suppressed.

Still further, air bag 10A having band-shaped connecting portion 12 of the above-described configuration contained in inflated/deflated space 15 is easy to fabricate, since it can be implemented with a simple configuration requiring only melting and bonding of three resin sheets. Therefore, a cuff for a blood pressure monitor ensuring stable avascularization performance can be fabricated in a simple and inexpensive manner.

### Second Embodiment

Fig. 7 is a schematic cross sectional view of an air bag of a cuff for a blood pressure monitor according to a second embodiment of the present invention, taken along the winding direction of the cuff. Fig. 8 is a schematic cross sectional view of the same air bag taken along the direction orthogonal to the winding direction of the cuff. The cuff for a blood pressure monitor of the present embodiment is identical to the cuff for a blood pressure monitor of the first embodiment except for the configuration of the air bag, and thus, the differences in structure of the air bag will be described in the following. As for the air bag as well, the corresponding portions are denoted by the same reference characters, and description thereof will not be repeated.

As shown in Figs. 7 and 8, in the air bag 10B of the cuff for a blood pressure monitor of the present embodiment, as in the case of air bag 10A of cuff 1A for a blood pressure monitor of the first embodiment, a band-shaped connecting portion 12 is arranged inside an inflated/deflated space 15 formed by an outer wall portion 11 a and an inner wall portion 11b. Band-shaped connecting portion 12 of the present embodiment, however, differs from the band-shaped connecting portion of the first embodiment in that it is formed of a stack of three resin sheets 13a, 13b and 13c stacked in the thickness direction.

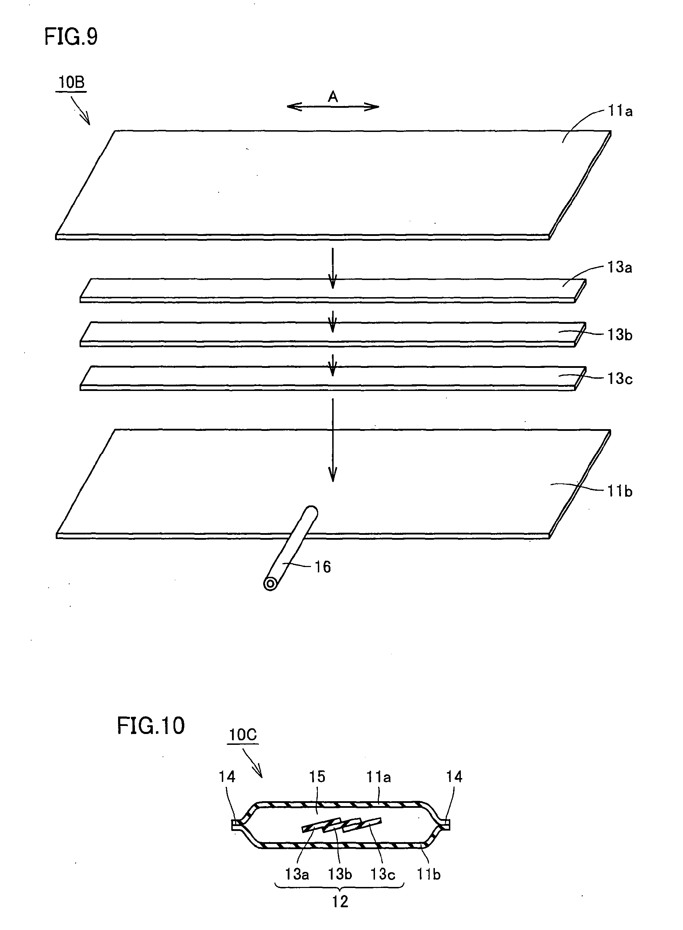

Fig. 9 is an exploded perspective view of the air bag of the cuff for a blood pressure monitor of the present embodiment. As shown in Fig. 9, in fabricating air bag 10B, a stack of three narrow resin sheets 13a, 13b, 13c constituting band-shaped connecting portion 12 is arranged in place between the two wide resin sheets constituting outer wall portion 11a and inner wall portion 11b. The five resin sheets in total are melted and bonded together at a pair of opposite sides located at the ends in the winding direction of air bag 10B. The two resin sheets constituting outer wall portion 11a and inner wall portion 11b are melted and bonded together at the remaining sides, with a tube 16 being inserted therebetween on one of these remaining sides. As such, the resin sheets constituting outer wall portion 11a and inner wall portion 11b, as well as the resin sheets constituting the stack serving as band-shaped connecting portion 12, are melted and bonded together in a single operation, so that air bag 10B is formed in an integral manner.

With such a configuration, even in the case where band-shaped connecting portion 12 is formed with the resin sheets of the same type (formed of the same material to have the same thickness) as the resin sheets constituting outer wall portion 11a and inner wall portion 11b, the thickness of band-shaped connecting portion 12 can be increased by the thicknesses of the stacked resin sheets. Accordingly, it is possible to ensure a wide cross sectional area of the air passages formed outside of the respective ends in the width direction of band-shaped connecting portion 12. As such, in addition to the effect obtained in the first embodiment, the effect of uniformly inflating air bag 10B over the entire region still more reliably can be obtained.

Fig. 10 shows a modification of the cuff for a blood pressure monitor of the present embodiment. Specifically, it shows a schematic cross sectional view of the air bag taken along the direction orthogonal to the winding direction of the cuff. As shown in Fig. 10, in air bag 10C of the present modification, band-shaped connecting portion 12 is formed of three resin sheets 13a, 13b, 13c stacked offset from each other in the width direction, and resin sheets 13a, 13b, 13c are contained inside inflated/deflated space 15 of air bag 10C while maintaining the state where they are inclined in the same direction.

With this configuration, the number of air passages can be increased, since separate air passages are formed outside of the respective ends in the width direction of resin sheets 13a, 13b and 13c constituting band-shaped connecting portion 12. Accordingly, in addition to the effect obtained in the first embodiment, the effect of uniformly inflating air bag 10C over the entire region still more reliably can be obtained.

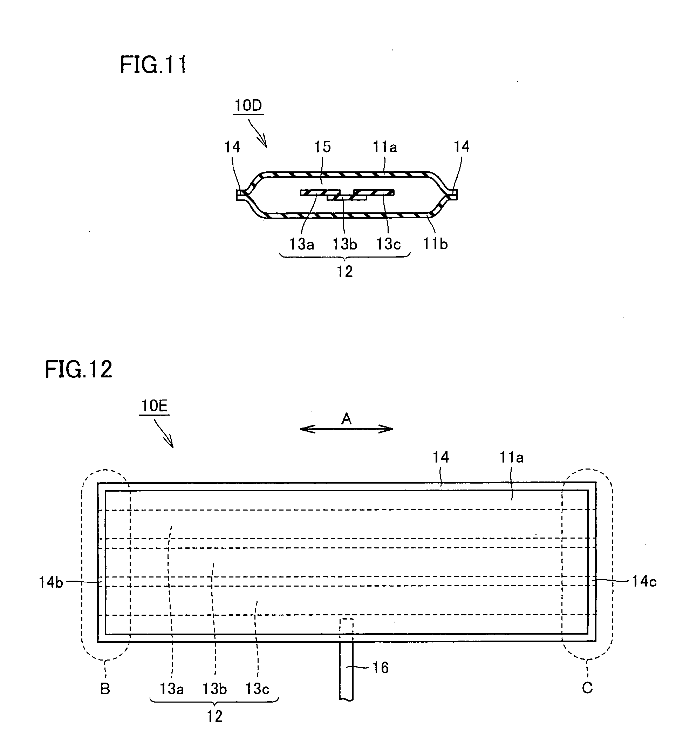

Fig. 11 shows another modification of the cuff for a blood pressure monitor of the present embodiment. Specifically, it shows a schematic cross sectional view of the air bag taken along the direction orthogonal to the winding direction of the cuff. As shown in Fig. 11, in the air bag 10D of the present modification, band-shaped connecting portion 12 is formed of three resin sheets 13a, 13b, 13c stacked offset from each other in the width direction, and resin sheets 13 a, 13b, 13c are contained inside inflated/deflated space 15 of air bag 10D while maintaining the state where they are horizontally arranged in an upper level and a lower level alternately.

With this configuration, the number of air passages can be increased, since separate air passages are formed outside of the respective ends in the width direction of resin sheets 13 a, 13b and 13 c constituting band-shaped connecting portion 12. Accordingly, in addition to the effect in the first embodiment, the effect of uniformly inflating air bag 10D over the entire region still more reliably can be obtained.

### Third Embodiment

Fig. 12 is a schematic top plan view showing a structure of an air bag of a cuff for a blood pressure monitor according to a third embodiment of the present invention. The cuff for a blood pressure monitor of the present embodiment is identical to the cuff for a blood pressure monitor of the first embodiment except for the configuration of the air bag. Thus, in the following, only the differences in structure of the air bag will be described. In the air bag as well, the corresponding portions are denoted by the same reference characters, and description thereof will not be repeated.

As shown in Fig. 12, in the air bag 10E of the cuff for a blood pressure monitor of the present embodiment, as in the case of air bag 10A of cuff 1A for a blood pressure monitor of the first embodiment, a band-shaped connecting portion 12 is arranged inside an inflated/deflated space 15 formed by an outer wall portion 11a and an inner wall portion 11b. However, band-shaped connecting portion 12 of the present embodiment differs from that of the first embodiment in that three resin sheets 13a, 13b and 13c are arranged in parallel with and spaced apart from each other in the direction orthogonal to the winding direction of the cuff.

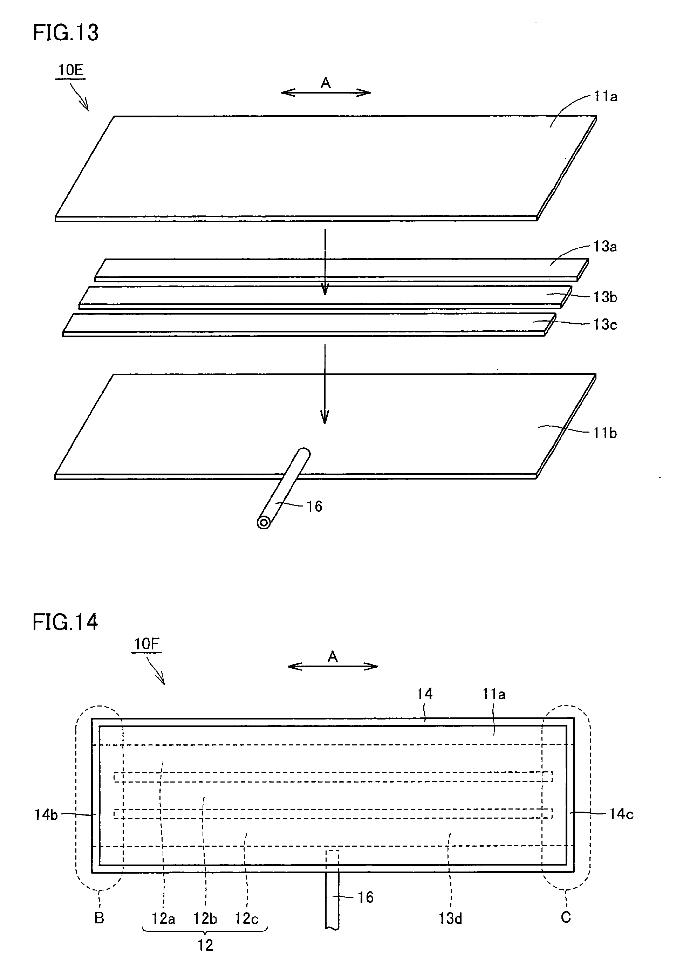

Fig. 13 is an exploded perspective view of the air bag of the cuff for a blood pressure monitor of the present embodiment. As shown in Fig. 13, in fabricating air bag 10E, three narrow resin sheets 13a, 13b and 13c constituting band-shaped connecting portion 12 are arranged in parallel with each other in place between two wide resin sheets constituting outer wall portion 11a and inner wall portion 11b. These five resin sheets in total are melted and bonded together at a pair of opposite sides located at the ends in the winding direction of air bag 10E. The two resin sheets constituting outer wall portion 11a and inner wall portion 11b are melted and bonded together at the remaining opposite sides, in one of which sides a tube 16 is inserted between the two sheets. As such, the resin sheets constituting outer wall portion 11a and inner wall portion 11b, as well as the three resin sheets arranged in parallel and constituting band-shaped connecting portion 12, are melted and bonded together in a single operation, and accordingly, air bag 10E is formed in an integral manner.

With such a configuration, the number of air passages can be increased, since separate air passages are formed on the outside of the respective ends in the width direction of resin sheets 13a, 13b, 13c constituting band-shaped connecting portion 12. Further, it is possible to arrange the plurality of air passages uniformly in the width direction of air bag 10E. As such, in addition to the effect of the first embodiment, the effect of uniformly inflating air bag 10E over the entire region still more reliably can be obtained.

### Fourth Embodiment

Fig. 14 is a schematic top plan view showing a structure of an air bag of a cuff for a blood pressure monitor according to a fourth embodiment of the present invention. The cuff for a blood pressure monitor of the present embodiment is identical to that of the first embodiment except for the configuration of the air bag, and therefore, only the differences in structure of the air bag will be described in the following. As for the air bag as well, the corresponding portions are denoted by the same reference characters, and description thereof will not be repeated.

As shown in Fig. 14, in the air bag 10F of the cuff for a blood pressure monitor of the present embodiment, as in the case of air bag 10A of cuff 1A for a blood pressure monitor of the first embodiment, a band-shaped connecting portion 12 is arranged in an inflated/deflated space 15 formed by an outer wall portion 11a and an inner wall portion 11b. However, band-shaped connecting portion 12 of the present embodiment differs from that of the first embodiment in that slits are provided in one resin sheet 13d to form three band-shaped connecting portions 12a, 12b and 12c arranged in parallel with and spaced apart from each other in the direction orthogonal to the winding direction of the cuff.

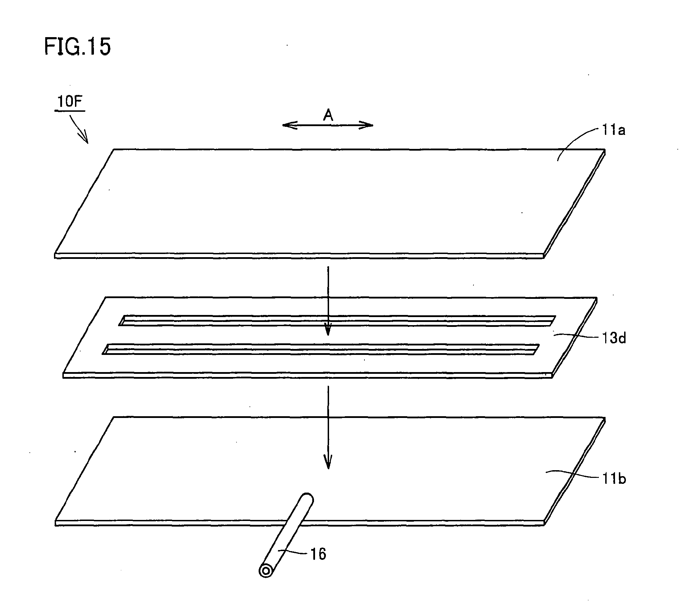

Fig. 15 is an exploded perspective view of the air bag of the cuff for a blood pressure monitor of the present embodiment. As shown in Fig. 15, in fabricating air bag 10F, one resin sheet 13d constituting band-shaped connecting portion 12 is arranged in place between two wide resin sheets constituting outer wall portion 11a and inner wall portion 11b, and the three resin sheets in total are melted and bonded together at a pair of opposite sides located at the ends in the winding direction of air bag 10F. At the remaining sides, the two resin sheets constituting outer and inner wall portions 11a and 11b are melted and bonded together, with a tube 16 being inserted therebetween at one of the relevant sides. As such, three resin sheets in total, including those constituting outer and inner wall portions 11 a and 11b and resin sheet 13 d constituting band-shaped connecting portion 12, are melted and bonded together in a single operation, to form air bag 10F in an integral manner.

With this configuration, the number of air passages can be increased, since separate air passages are formed on the outside of the respective ends in the width direction of band-shaped connecting portions 12a, 12b and 12c. Further, the air passages can be arranged uniformly in the width direction of air bag 10F. Accordingly, in addition to the effect obtained in the first embodiment, the effect of uniformly inflating air bag 10F over the entire region still more reliably can be obtained.

### Fifth Embodiment

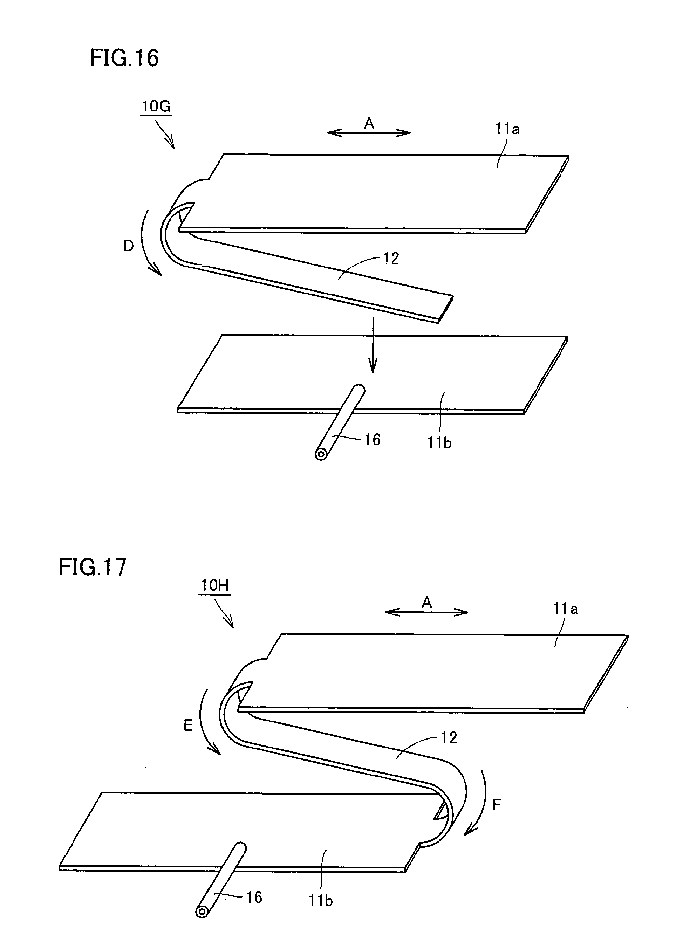

Fig. 16 is an exploded perspective view of an air bag of a cuff for a blood pressure monitor according to a fifth embodiment of the present invention. The cuff for a blood pressure monitor of the present embodiment is identical to that of the first embodiment except for the configuration of the air bag. Thus, in the following, only the differences in structure of the air bag will be described. In the air bag as well, the corresponding portions are denoted by the same reference characters, and description thereof will not be repeated.

In the air bag 10G of the cuff for a blood pressure monitor of the present embodiment, as in the case of air bag 10A of cuff 1A for a blood pressure monitor of the first embodiment, a band-shaped connecting portion 12 is arranged in an inflated/deflated space 15 formed by an outer wall portion 11a and an inner wall portion 11b. However, as shown in Fig. 16, band-shaped connecting portion 12 in air bag 10G of the cuff for a blood pressure monitor of the present embodiment differs from the band-shaped connecting portion of the first embodiment in that, prior to melting and bonding of air bag 10G, it is formed of a narrow resin sheet portion that continuously extends from one end in the longitudinal direction of a wide resin sheet portion constituting outer wall portion 111a.

As shown in Fig. 16, in fabricating air bag 10G, the resin sheet portion constituting band-shaped connecting portion 12, formed to extend from an end of the resin sheet portion constituting outer wall portion 11 a, is folded back in the direction shown by an arrow D in the figure at the boundary of the resin sheet portions, toward a main surface of the resin sheet portion constituting outer wall portion 11a. With this state being maintained, the resin sheet is laid on another resin sheet constituting inner wall portion 11b, such that the folded-back resin sheet portion constituting band-shaped connecting portion 12 is arranged between the resin sheet portion constituting outer wall portion 11a and the other resin sheet constituting inner wall portion 11b. The resin sheets are melted and bonded together at a pair of opposite sides located at the ends in the winding direction of air bag 10G. The resin sheet portion constituting outer wall portion 11 a and the resin sheet constituting inner wall portion 11b are melted and bonded together at the remaining sides, in one of which sides a tube 16 is inserted therebetween. As such, the resin sheet constituting outer wall portion 11a and band-shaped connecting portion 12 and the resin sheet constituting inner wall portion 11b are melted and bonded together in a single operation, to form air bag 10G in an integral manner.

With this configuration, in addition to the effect obtained in the first embodiment, another effect is obtained that air bag 10G having band-shaped connecting portion 12 contained in inflated/deflated space 15 can readily be fabricated using two resin sheets.

### Sixth Embodiment

Fig. 17 is an exploded perspective view of an air bag of a cuff for a blood pressure monitor according to a sixth embodiment of the present invention. The cuff for a blood pressure monitor of the present embodiment is identical to that of the first embodiment except for the configuration of the air bag, and therefore, only the differences in structure of the air bag will be described in the following. As for the air bag as well, the corresponding portions are denoted by the same reference characters, and description thereof will not be repeated.

In the air bag 10H of the cuff for a blood pressure monitor of the present embodiment, as in the case of air bag 10A of cuff 1A for a blood pressure monitor of the first embodiment, a band-shaped connecting portion 12 is arranged in an inflated/deflated space 15 formed by an outer wall portion 11 a and an inner wall portion 11b. However, in air bag 10H of the cuff for a blood pressure monitor of the present embodiment, as shown in Fig. 17, prior to melting and bonding of air bag 10H, band-shaped connecting portion 12 is formed of a narrow resin sheet portion that continuously extends from one end in the longitudinal direction of a wide resin sheet portion constituting outer wall portion 11a, and inner wall portion 11b is formed of a wide resin sheet portion that continuously extends from the other end in the longitudinal direction of the narrow resin sheet portion constituting band-shaped connecting portion 12. That is, outer wall portion 11a and inner wall portion 11b of air bag 10H as well as band-shaped connecting portion 12 are formed from one, non-divided resin sheet.

As shown in Fig. 17, in fabricating air bag 10H, the resin sheet portion constituting band-shaped connecting portion 12, having been formed to extend from one end of the resin sheet portion constituting outer wall portion 11a, is folded back at the boundary between these resin sheet portions in the direction shown by an arrow E in the figure, and the resin sheet portion constituting inner wall portion 11b, having been formed to extend from the other end of the resin sheet portion constituting band-shaped connecting portion 12, is folded back at the boundary between these resin sheet portions in the direction shown by an arrow F in the figure, such that the resin sheet portion constituting band-shaped connecting portion 12 is arranged between the resin sheet portion constituting outer wall portion 11a and the resin sheet portion constituting inner wall portion 11b. The resin sheet portions laid in this manner are melted and bonded together at a pair of opposite sides located at the ends in the winding direction of air bag 10H. At the remaining sides, the resin sheet portions constituting outer and inner wall portions 11a and 11b are melted and bonded together, with a tube 16 being inserted therebetween at one of the remaining sides. As such, the resin sheet portions constituting outer wall portion 11a, inner wall portion 11b and band-shaped connecting portion 12 are melted and bonded together in a single operation, to form air bag 10H in an integral manner.

With this configuration, in addition to the effect obtained in the first embodiment, another effect is obtained that air bag 10H having band-shaped connecting portion 12 arranged inside inflated/deflated space 15 can readily be fabricated using one resin sheet.

### Seventh Embodiment

Fig. 18 is an exploded perspective view of an air bag of a cuff for a blood pressure monitor according to a seventh embodiment of the present invention. The cuff for a blood pressure monitor of the present embodiment is identical to that of the first embodiment except for the configuration of the air bag, and therefore, only the differences in structure of the air bag will be described in the following. As for the air bag as well, the corresponding portions are denoted by the same reference characters, and description thereof will not be repeated.

In the air bag 10I of the cuff for a blood pressure monitor of the present embodiment, as in the case of air bag 10A of cuff 1A for a blood pressure monitor of the first embodiment, a band-shaped connecting portion 12 is arranged in an inflated/deflated space 15 formed by an outer wall portion 11a and an inner wall portion 11b. However, in air bag 10I of the cuff for a blood pressure monitor of the present embodiment, as shown in Fig. 18, prior to melting and bonding of air bag 10I, inner wall portion 11b is formed of a wide resin sheet portion that continuously extends from one end in the longitudinal direction of a wide resin sheet portion constituting outer wall portion 11a, and band-shaped connecting portion 12 is formed of a narrow resin sheet portion that continuously extends from the other end in the longitudinal direction of the wide resin sheet portion constituting inner wall portion 11b. That is, outer wall portion 11a and inner wall portion 11b of air bag 10I and band-shaped connecting portion 12 are formed of one, non-divided resin sheet.

As shown in Fig. 18, in fabricating air bag 10I, the resin sheet portion constituting band-shaped connecting portion 12, having been formed to extend from one end of the resin sheet portion constituting inner wall portion 11b, is folded back at the boundary between these resin sheet portions in the direction shown by an arrow G in the figure, and the resin sheet portion constituting outer wall portion 11a, having been formed to extend from the other end of the resin sheet portion constituting inner wall portion 11b, is folded back at the boundary between these resin sheet portions in the direction shown by an arrow H in the figure, such that the resin sheet portion constituting band-shaped connecting portion 12 is arranged between the resin sheet portions constituting outer and inner wall portions 11a and 11b. The resin sheet portions thus laid are melted and bonded together at a pair of opposite sides located at the ends in the winding direction of air bag 10I. The resin sheet portions constituting outer and inner wall portions 11a and 11b are melted and bonded together at the remaining sides, with a tube 16 being inserted therebetween at one of the remaining sides. As such, the resin sheet portions constituting outer wall portion 11a, inner wall portion 11b and band-shaped connecting portion 12 are melted and bonded together in a single operation, to form air bag 10I in an integral manner.

With this configuration, in addition to the effect obtained in the first embodiment, another effect is obtained that air bag 10I having band-shaped connecting portion 12 arranged inside inflated/deflated space 15 can readily be fabricated using one resin sheet.

In the first through seventh embodiments described above, the cuff for a blood pressure monitor having the air bag identified as the fluid bag that is in an approximately rectangular shape in two dimensions has been described by way of example. The shape of the air bag, however, is not restricted thereto. It may be in an elliptic shape, or may be curved at ends in the longitudinal direction. The present invention is applicable to the air bag of any shape.

Further, in the cuff for a blood pressure monitor in each of the first through seventh embodiments above, the air bag identified as the fluid bag has been formed by melting and bonding resin sheets together. The way of joining the resin sheets, however, is not restricted to such melting and bonding. For example, the resin sheets may be adhered to each other. Further, the material of the sheet-shaped members is not restricted to resin, but other materials may also be employed.

Still further, in the first through seventh embodiments described above, the case of applying the present invention to the cuff for use in a wrist blood pressure monitor assuming a wrist as the measurement site has been explained by way of example. However, the present invention is applicable to the cuff for a blood pressure monitor of any type, such as the one fitted to an upper arm, the one fitted to a finger, or the like.

Although the present invention has been described and illustrated in detail, it is clearly understood that the same is by way of illustration and example only and is not to be taken by way of limitation, the spirit and scope of the present invention being limited only by the terms of the appended claims.

## Claims

1. A cuff for a blood pressure monitor wound around a living body, comprising a fluid bag (10A-10I) having an inflated/deflated space (15) permitting a fluid to come in/out formed therein by joining rims of sheet-shaped members laid one on another, wherein
said fluid bag (10A-10I) includes a band-shaped connecting portion (12) arranged inside said inflated/deflated space (15), said band-shaped connecting portion (12) extending substantially continuously from one end (B) to the other end (C) of the fluid bag (10A-10I) in a winding direction (A) along which the fluid bag (10A-10I) is wound around the living body, and having ends respectively attached to said one end (B) and said other end (C) of said fluid bag (10A-10I).

2. The cuff for a blood pressure monitor according to claim 1, wherein a plurality of said band-shaped connecting portions (12) are arranged inside said inflated/deflated space (15).

3. The cuff for a blood pressure monitor according to claim 1, wherein
said sheet-shaped members and said band-shaped connecting portion (12) are formed of resin materials that can be melted and bonded together, and
joining of the rims of said sheet-shaped members and attaching of said band-shaped connecting portion (12) to said fluid bag (10A-10I) are carried out by melting and bonding the rims of said sheet-shaped members and said band-shaped connecting portion together in an integral manner.

4. The cuff for a blood pressure monitor according to claim 1, wherein
said sheet-shaped members and said band-shaped connecting portion (12) are formed of a same resin material, and
joining of the rims of said sheet-shaped members and attaching of said band-shaped connecting portion (12) to said fluid bag (10A-10I) are carried out by melting and bonding the rims of said sheet-shaped members and said band-shaped connecting portion together in an integral manner.

5. The cuff for a blood pressure monitor according to claim 1, wherein
said fluid bag (10G) includes an inner wall portion (11b) positioned on an inner side and an outer wall portion (11a) positioned on an outer side in a fitted state of the cuff,
said sheet-shaped members are configured with a first resin sheet having said band-shaped connecting portion (12) and one of said inner wall portion (11b) and said outer wall portion (11a), and a second resin sheet having the other of said inner wall portion (11b) and said outer wall portion (11a), and said fluid bag (10G) is formed by folding the first resin sheet, laying the first resin sheet on the second resin sheet, and by melting and bonding rims of the first and second resin sheets together.

6. The cuff for a blood pressure monitor according to claim 1, wherein
said fluid bag (10H, 10I) includes an inner wall portion (11b) positioned on an inner side and an outer wall portion (11a) positioned on an outer side in a fitted state of the cuff,
said sheet-shaped members are configured with one resin sheet having said inner wall portion (11b), said outer wall portion (11a) and said band-shaped connecting portion (12), and
said fluid bag (10H, 10I) is formed by folding the resin sheet such that said inner and outer wall portions are laid one on another, with said band-shaped connecting portion arranged therebetween, and by melting and bonding rims of said inner and outer wall portions of the resin sheet together.
